# EUROPEAN PATENT APPLICATION

(11) **EP 2 896 699 A1**
(43) Date of publication of application: **22.07.2015**
(21) Application number: 13837622.3
(22) Date of filing: 11.09.2013
(51) Int. Cl.: C12P 7/06, C12P 7/10, C12P 19/12

(54) **METHOD FOR PRODUCING SUGAR AND ETHANOL BY SELECTIVE FERMENTATION**

(30) Priority: 14.09.2012 JP 2012203266
(71) Applicant: Asahi Group Holdings, Ltd., Tokyo 130-8602 (JP)
(72) Inventor: OHARA, Satoshi, Moriya-shi Ibaraki 302-0106 (JP)
(74) Representative: Schnappauf, Georg
(86) International application number: PCT/JP2013/074519
(87) International publication number: WO 2014/042184

(57) **Abstract**

[Object] An object of the present invention is to provide a method for producing sugar and ethanol wherein sucrose is hardly decomposed during fermentation of a sugar liquid; yield of the sugar is high; and at the same time, yield of the ethanol is high.

[Solving Means] A method for producing sugar and ethanol comprising the steps of: heating and cleaning a sugar liquid derived from a plant; fermenting the cleaned liquid which has been adjusted to suitable temperature to selectively convert sugar components other than sucrose in the cleaned liquid into ethanol; and concentrating the obtained fermented liquid.

## Description

### [Technical Field]

The present invention relates to a method for producing sugar and ethanol, and more specifically, relates to a method for producing sugar and ethanol wherein a sugar liquid derived from a plant is fermented.

### [Background Art]

Ethanol for fuel derived from a plant is expected to be a liquid fuel alternative to gasoline to prevent increase in carbon dioxide gas, and a method for producing ethanol by fermenting a sugar liquid derived from a plant with a microorganism has been conventionally investigated. However, there is a problem that consumption of a sugar liquid derived from a plant as a raw material for production of ethanol puts pressure on production of sugar, which is a food.

As a method to solve this problem, Patent Document 1 describes a method for producing sugar and ethanol which can cover almost all of the energy consumed in, for example, a production process of sugar and ethanol by the energy obtained by burning a squeezed residue from sugar cane without causing decrease in the amount of sugar.

In addition, Patent Document 2 describes a method wherein a sugar liquid derived from a plant is, by first, fermented with yeast having no sucrose-degrading enzyme, the fermented liquid is cleaned with heating and filtering, the cleaned sugar liquid is concentrated to separate ethanol contained in the sugar liquid after fermentation, and then, sucrose is crystallized to produce sugar and ethanol, in order to further improve production efficiency of sugar and ethanol. The method is characterized in that a concentration step which has conventionally been utilized for evaporating water content in a sugar liquid is, at the same time, utilized for evaporating ethanol, when conventional sugar producing steps are conducted.

A sugar liquid derived from a plant, for example, sugar cane squeezed juice and the like, has a sugar concentration and a temperature suitable for conducting ethanol fermentation with yeast. A sugar liquid derived from a plant, for example, sugar cane squeezed juice and the like is, generally, heated by first, sterilization of microorganisms derived from a raw material and deposition of protein in the sugar liquid are then conducted, and through a cleaning step of precipitating and separating foreign substances by incorporating additives such as lime or an agglomerating and precipitating agent, and, thereafter, utilized for producing sugar and ethanol. The temperature of the sugar liquid after the cleaning step rises to high level which is not suited for conducting ethanol fermentation. The process of ref. 2 is, thus, characterized in that the fermentation step is conducted prior to the cleaning step.

In the method of Patent Document 2, however, a sugar liquid derived from a plant to be fermented has not been heated nor sterilized. So, when the fermentation time is prolonged in a sugar liquid containing a large amount of invert sugar, it becomes large the amount of sucrose decomposed by incorporation of the microorganisms other than yeast during fermentation of the sugar liquid, and it is difficult to increase the yield of sugar. In addition, the microorganisms can convert the decomposed sugar components into other substances such as lactic acid, acetic acid or the like. Therefore, there is a limit on increasing the yield of ethanol. In addition, since a sugar liquid derived from a plant generally contains large amount of foreign substances, microorganisms and the like, it is difficult to repeatedly utilize yeast, and an efficient fermentation method wherein especially a cohesive yeast is always present in a fermenter to continuously carry out fermentation without separation of the yeast is difficult. Additionally, there is a problem that, when the heated fermented liquid is stood still in a precipitation tank in the cleaning step after fermentation, a part of heated alcohol is evaporated, and the final amount of recovery of ethanol decreases since the precipitation tank is generally the open air type.

Patent Document 3 describes that glucose in an aqueous solution of the substrate containing sucrose and fructose polymers is selectively subjected to ethanol fermentation with using yeast capable of fermenting glucose to alcohol, but which does not hydrolyze fructose polymers or sucrose. The substrate containing sucrose and fructose polymers has been prepared by simultaneous action of fructosyl transferase and glucose isomerase on a sucrose containing substrate. As the sucrose containing substrate, molasses and the like are illustrated.

The invention of Patent Document 3 aims at providing sweet syrup high in fructose content using molasses and the like as raw materials. The molasses mean the residue obtained after sugar is crystallized and recovered from a sugar liquid, that is, the residue obtained from a conventional sugar producing method. The invention of Patent Document 3 does not pertains to a process for utilizing conventional sugar producing steps such as those of Patent Document 2, and the objective product is also different. The syrup high in fructose content is low in sucrose content, and the invention of Patent Document 3 consumes not only glucose, but also sucrose.

The present invention aims at improving the yield of sugar which is sucrose crystal, and relates to technics for improving the pure sugar rate of sugar liquid, that is, content ratio of sucrose that occupies in all the soluble solid components, to improve recovery efficiency of sugar crystal by the selective fermentation of glucose and fructose. Therefore, the invention of

Patent Document 3 is distinguished in objective from the present invention.

### [Background Art Documents]

### [Patent Documents]

[Patent Document 1] Japanese Patent Laid-open Publication No. 2004-321174
[Patent Document 2] Japanese Patent No. 4883511
[Patent Document 3] US Patent No. 4,335,207

### [Summary of the Invention]

### [Problems to be Solved by the Invention]

The present invention solves the above-mentioned conventional problems, and an objective thereof is to provide a method for producing sugar and ethanol by utilizing conventional sugar producing steps, wherein sucrose is not decomposed during fermentation of a sugar liquid; recover amount of the sugar is improved; and at the same time, recover amount of the ethanol is improved.

### [Means for Solving the Problems]

The present invention provides a method for producing sugar and ethanol comprising the steps of:
heating and cleaning a sugar liquid derived from a plant;
fermenting the cleaned liquid which has been adjusted to suitable temperature to selectively convert sugar components other than sucrose in the cleaned liquid into ethanol; and
concentrating the obtained fermented liquid.

In one embodiment, the fermentation is carried out using a yeast having no sucrose-degrading enzyme.

In one embodiment, the fermentation is carried out using a yeast of which sucrose-degrading enzyme gene is disrupted.

In one embodiment, the fermentation is carried out in the presence of a sucrose-degrading enzyme inhibitor.

In one embodiment, a crop as a raw material of the sugar is at least one kind selected from the group consisting of sugar cane, sugar beet, sugar palm, sugar maple and sorghum.

In one embodiment, the cleaned liquid has a sucrose concentration of not less than 9% by weight.

In one embodiment, the fermented liquid has a pure sugar rate of not less than 50%.

In one embodiment, the concentration is conducted by distilling the fermented liquid under reduced pressure, and thereby, ethanol is separated and recovered from the fermented liquid.

In one embodiment, the method for producing sugar and ethanol further comprising the step of crystallizing sugar from the concentrated sugar liquid obtained by concentrating the fermented liquid.

### [Effects of the Invention]

By means of the method of the present invention, since fermentation is carried out using a heated and cleaned sugar liquid, even when the fermentation time is prolonged in the sugar liquid containing a large amount of invert sugar, sucrose is hardly decomposed during fermentation of the sugar liquid; yield of the sugar is high; and at the same time, yield of the ethanol is high. In addition, since the sugar liquid to be subjected to fermentation has been subjected to inactivation of microorganisms by heating and cleaned by removing foreign substances, it hardly occurs that the yeast is contaminated by incorporation of microorganisms or foreign substances, and recovery and reuse of the yeast can be easily carried out. Furthermore, microorganisms or foreign substances are not accumulated in a fermentation tank as the cleaned liquid is utilized, a yeast separator becomes unnecessary since a yeast having a cohesive property becomes available, thereby shortening of process time becomes possible. Additionally, since concentration is directly carried out without using a precipitation tank after fermentation, loss of ethanol through evaporation at a settlement tank can also be avoided.

### [Brief Description of the Drawings]

[Fig. 1] A flow diagram of the process used in Example 1.
[Fig. 2] A diagram showing material balance of the process of Example 1.
[Fig. 3] A diagram showing material balance of the process of Comparative Example 1.

### [Mode for Carrying out the Invention]

In the method of the present invention, the plants to be used as a raw material of the sugar liquid derived from a plant are plants which can accumulate sugar components. Among them, a so-called crop as a raw material of sugar is preferable. Specific examples of the crop as a raw material of sugar include sugar cane, sugar beet, sugar palm, sugar maple, sorghum and the like. Especially preferable plants are sugar cane and sugar beet. This is because the plants accumulate a large amount of sugar components. There are sugar producing factories employing them as raw materials, and such sugar producing factories can easily incorporate the present invention.

The sugar liquid derived from a plant refers to a liquid obtained by extracting a component containing sugar components from a plant. The sugar liquid derived from a plant generally includes a squeezed juice obtained by compressing a part in which the sugar components of the plant is accumulated, a broth prepared by decocting the part in which sugar components of the plant is accumulated, and the like.

Usually, the plant is cut or crushed into an appropriate size before being compressed or decocted. A means for squeezing a juice such as a roll mill or the like may be used for compression of a plant. In addition, upon decocting a plant, decoction means such as a diffuser may be used. The temperature of the filling water as being compressed and the decoction temperature may be appropriately determined taking extraction efficiency of sugar components and the like into consideration. The temperature is usually 30 to 40°C.

In order to inactivate a sucrose-degrading enzyme, and to modify, deposit and precipitate proteins and the like in the sugar liquid, heating is carried out. Heating temperature is 65 to 105°C, and preferably 80 to 105°C. When the heating temperature is lower than 65°C, a sucrose-degrading enzyme cannot be inactivated during fermentation of the sugar liquid. Here, several seconds to 10 minutes of heating time is sufficient for inactivating a sucrose-degrading enzyme. In addition, when the heating temperature is lower than 65°C, sterilization of the sugar liquid is insufficient. In order to sufficiently carry out sterilization of the sugar liquid, it is preferable to adjust the heating temperature to 100°C or higher.

Optimum conditions of the heating in the cleaning step differ depending on the scale of implementation and the like. In an actual production process, it is preferable to carry out heating for several hours for precipitating impurities in the sugar liquid. The heating time for precipitating the impurities in the sugar liquid is 2 to 4 hours, and preferably about 3 hours. When the heating time is less than 2 hours, it is difficult to precipitate the impurities in the sugar liquid.

Cleaning of the sugar liquid refers to removal of solid components other than sucrose contained in the sugar liquid. The solid components other than sucrose include insoluble solid components such as cellulose, hemicellulose and the like; and soluble solid components such as protein, pectin, amino acid, organic acid, reducing sugar, ash and the like.

Removal of the solid components other than sucrose is carried out, for example, in the following way. First, lime is added to the sugar liquid which has been heated, to aggregate protein, pectin and the like. If needed, calcium hydroxide or calcium oxide is added thereto, or carbon dioxide gas is blown thereto to produce calcium carbonate, by which an aggregate of the non-sugar components is adsorbed to calcium carbonate and precipitated. Next, an insoluble component containing the aggregate and the precipitate is separated by filtration, to give a cleaned liquid. The cleaned liquid mainly contains sucrose, glucose, fructose and the like.

The cleaned liquid is a cleaned sugar liquid, and is an aqueous solution having a sucrose concentration of not less than 9% by weight, preferably 9 to 18% by weight, more preferably 12 to 15% by weight. If the sucrose concentration is less than 9% by weight, the recovery amount of sugar may become poor. This is because the sugar concentration in a concentrated liquid becomes less than 50% by weight when conventional concentration apparatuses in a sugar producing process, for example, a fivefold effect evaporator, are employed, and sugar crystalline can be fused in the crystallization step. The cleaned liquid has a pure sugar rate of not less than 50%.

Next, the cleaned liquid is cooled, left or optionally heated to adjust a temperature suitable for conducting fermentation. The temperature suitable for conducting fermentation is 10 to 50°C, preferably 20 to 40°C, more preferably 25 to 35°C. The cleaned liquid which has been adjusted to suitable temperature is fermented, to selectively convert the sugar components other than sucrose in the cleaned liquid into ethanol. Such a concept of selective fermentation method is disclosed in Japanese Patent No. 4883511.

As a result of the selective fermentation, the content of sugar components other than sucrose becomes quite low. The content of invert sugar can become zero dependent on conditions of the selective fermentation. The selective fermentation decreases the invert sugar concentration and decreases the soluble solid component concentration, while keeps the sucrose amount constant in the cleaned liquid, and, therefore, the pure sugar rate improves. The cleaned liquid after completion of the selective fermentation has a pure sugar rate of not less than 70%, preferably not less than 80%, more preferably not less than 90%.

Pure sugar rate refers to % by weight of sucrose contained in soluble solid components (Brix) of the liquid.

One means for selective fermentation is fermentation carried out using a yeast having no sucrose-degrading enzyme.

Examples of the yeast having no sucrose-degrading enzyme include Saccharomyces cerevisiae ATCC56805, STX347-1D strain, Saccharomyces aceti NBRC10055, Saccharomyces hienipiensis NBRC1994, Saccharomyces italicus ATCC13057, Saccharomyces dairenensis NBRC 0211, Saccharomyces transvaalensis NBRC 1625, Saccharomyces rosinii NBRC 10008, Zygosaccharomyces bisporus NBRC 1131 and the like. In addition, even in a microorganism having a sucrose-degrading enzyme, a strain in which all or a part or the 6 kinds of sucrose-degrading enzyme genes possessed by a microorganism (SUC1, SUC2, SUC3, SUC4, SUC6 and SUC7) is disrupted by genetic engineering can also be used.

Another means for selective fermentation is fermentation carried out using a sucrose-degrading enzyme inhibitor.

Examples of the sucrose-degrading enzyme inhibitor include a silver ion, a copper ion, a mercury ion, a lead ion, methy-α-D-glucopyranoside, PCMB (p-chloromercuribenzoate), glucosyl-D-psicose and the like.

Operation and conditions for fermenting the cleaned liquid can be carried out by a method known to one skilled in the art, and include, for example, a batch method wherein fermentation is carried out by adding a fermentation microorganism and a sugar liquid in a given ratio, a continuous method wherein fermentation is carried out by immobilizing a fermentation microorganism and thereafter continuously feeding a sugar liquid, and the like.

However, in the method of the present invention, since inactivation of a microorganism and removal of a foreign substance are carried out by the above-mentioned cleaning step, sucrose degradation by a microorganism such as a wild yeast, a lactobacillus, an acetobacter or the like is not generated upon fermentation. In addition, production of a product other than ethanol (for example, lactic acid, acetic acid or the like) from invert sugar is prevented. Therefore, ethanol fermentation can be carried out with high efficiency. The yeast obtained after the cleaned liquid is fermented, does not contain microorganisms or foreign substances because of removal of microorganisms and foreign substances at the cleaning step, and the yeast after fermentation can repeatedly be utilized. Unlike molasses, the cleaned liquid can be utilized as a fermentation raw material without being diluted, and the water amount necessary for fermentation can be reduced.

The amount of the yeast upon fermenting the cleaned liquid is 5 g/L or more, preferably 10 to 100 g/L, and more preferably 15 to 60 g/L in wet weight. An amount of the yeast to be added of less than 5 g/L does not progress fermentation, and an excessively large amount causes inefficient separation of the liquid from the yeast upon recovery of the yeast.

The fermented liquid obtained as a result of the fermentation contains the yeast, ethanol, water, sucrose, mineral, amino acid and the like. After completion of fermentation, the yeast are separated.

Next, the fermented liquid from which the yeast has been separated is concentrated to obtain a concentrated sugar liquid. The concentration is carried out for recovering ethanol, followed by evaporating water, and the fermented liquid is concentrated to produce sugar from the fermented liquid.

The concentrated sugar liquid has a pure sugar rate of not less than 85%, preferably not less than 90%, more preferably not less than 95%. The high pure sugar rate of the concentrated liquid makes the contained sucrose easy to crystallize, and the yield of sugar increases.

The recovery of ethanol from the fermented liquid, from which the yeast has been separated, can be carried out by a method known to one skilled in the art, and the method is, for example, separation of ethanol by distillation. Specifically, a distillation procedure utilizing a multifold effect evaporator of sugar producing factory is able to separate ethanol at the first effect evaporator and can evaporate water at the second evaporator or later, and, at the same time, the fermentation liquid is able to change into the concentrated sugar liquid. Thus, it is unnecessary to carry out heat concentration once again in production of sugar, and both time and energy can be saved.

The production of sugar from the concentrated liquid can be carried out by a method known to one skilled in the art, and the method is, for example, crystallization of sugar, or the like. Specifically, a part of the concentrated sugar liquid is heated under suction reduced pressure, while the residual sugar liquid is little by little added so that a supersaturation degree of 1.1 to 1.2 is maintained to let sugar crystal grow large. After the sugar crystal having a size of a given size or larger, the concentrate is then separated into a sugar crystal and a sugar liquid with a centrifuge machine.

The sugar liquid separated from the sugar crystal is generally referred to as molasses. The molasses may be mixed with the cleaned liquid in an appropriate amount, to be used again as a fermentation raw material. Thus, the utilization efficiency of the sugar components contained in the sugar liquid is further improved.

### [Examples]

Although the present invention will be explained more specifically by means of the examples described below, the present invention is never limited thereto.

### Example 1

(Demonstration of the process of fermenting a cleaned liquid in a case where a yeast having no sucrose-degrading enzyme is used, using sugar cane as a raw material)

### (1) Compressing step

Three thousand grams of stalk portions of sugar cane (NiF8) after harvesting were cut with a shredder, and thereafter compressed with a quadruple roll mill, to give 2,843 mL of a squeezed juice (weight of the squeezed juice = 2,985 g, sucrose content = 351 g, invert sugar content = 112 g, pure sugar rate = 63.9%).

### (2-1) Heating and cleaning step

The squeezed juice was transferred to a 5 L-beaker, and heated at 100°C for 10 minutes. Next, 0.085% by weight of slaked lime Ca(OH)₂ based on the weight of the squeezed juice was added thereto, to adjust pH and aggregate impurities. The aggregated impurities were filtrated with a filter, to separate 2,684 mL of a cleaned liquid (weight of the cleaned liquid = 2,818 g, sucrose content = 346 g, invert sugar content = 111 g, pure sugar rate = 71.7%). The contained impurities, which were 102 g in the squeezed juice, were decreased to 26 g in the cleaned liquid. Furthermore, in the cleaned liquid, the microorganism contained in the squeezed juice was sterilized by heating.

### (2-2) Fermentation step

The obtained cleaned liquid was transferred to a 5 L-jar fermenter and cooled to 30°C, and thereafter 134 g in wet weight of a cohesive yeast Saccharomyces cervisiae (STX347-1D) having no sucrose-degrading enzyme was inoculated thereto. Ethanol fermentation was carried out at 30°C under an anaerobic condition for 24 hours. The yeast had been previously precultured in a YM medium, and used. After completion of fermentation, total amount of 145 g of the yeast and aggregated impurities were recovered by precipitation separation, to separate 2,748 g of a fermented liquid (ethanol concentration: 1.75% by weight, sucrose content = 346 g, invert sugar content = 11 g).

### (3) Ethanol distillation and sugar liquid concentration step

The fermented liquid was heated under reduced pressure, and 46 g of evaporated ethanol was cooled and recovered. Thereafter, 2,073 mL of water was evaporated, to give 630 g of a concentrated sugar liquid (sucrose content = 346 g, invert sugar content = 11 g, pure sugar rate = 91.6%).

### (4) Crystallization step

A half of the sugar liquid was drawn out and further heated under reduced pressure, and concentrated to a degree of supersaturation of sucrose of 1.2. Thereafter, 32 g of seed crystals of sugar (particle size: 250 µm) were added thereto, and crystallization was carried out for about 3 hours, with adding the residual concentrated sugar liquid in small portions.

### (5) Raw sugar - molasses separation step

A mixture of the crystallized sugar and molasses was centrifuged in a perforated wall type centrifuge using a filter fabric with 50 to 100 µm mesh at 3,000 rpm for 20 minutes, to separate 253 g of sugar (sucrose recovery rate = 71%: excluding the amount of the added seed crystal) and 137 g of molasses (sucrose content = 100 g, invert sugar content = 8 g, pure sugar rate = 80.6%).

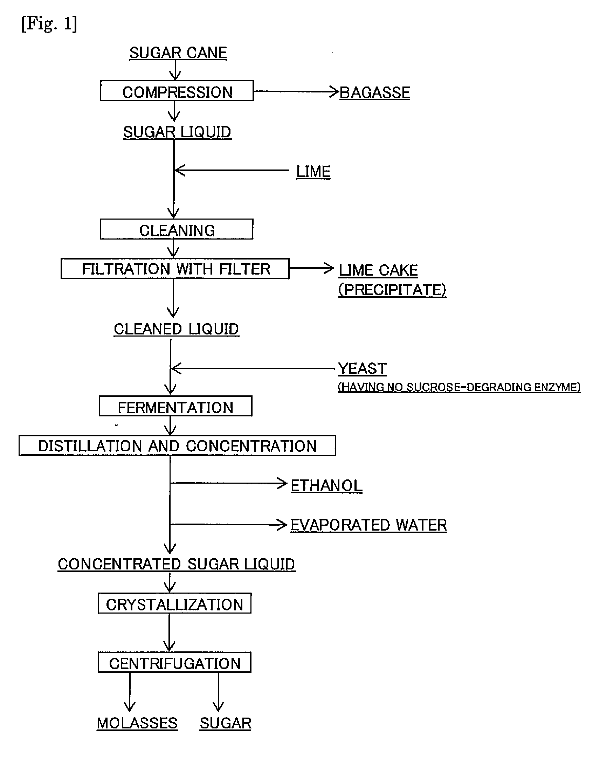

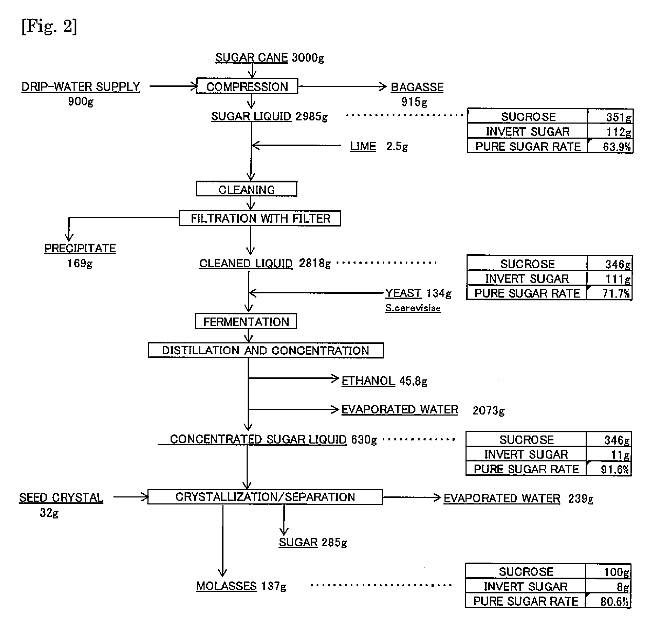

A flow diagram of the production process is shown in Fig. 1, and the result of the material balance is shown in Fig. 2.

### Comparative Example 1

(Demonstration of the process of fermenting a squeezed juice before the cleaning step in a case where a yeast having no sucrose-degrading enzyme is used, using sugar cane as a raw material)

### (1) Compressing step

Three thousand grams of stalk portions of sugar cane (NiF8) after harvesting were cut with a shredder, and thereafter compressed with a quadruple roll mill, to give 2,843 mL of a squeezed juice (weight of the squeezed juice = 2,985 g, sucrose content = 351 g, invert sugar content = 112 g, pure sugar rate = 63.9%).

### (2-1) Fermentation step

The obtained squeezed juice was transferred to a 5 L-jar fermenter, and thereafter 142 g in wet weight of a cohesive yeast Saccharomyces cervisiae (STX347-1D) having no sucrose-degrading enzyme was inoculated thereto. Ethanol fermentation was carried out at 30°C under an anaerobic condition for 24 hours. The yeast had been previously precultured in a YM medium, and used. After completion of fermentation, total amount of 245 g of the yeast and aggregated impurities were recovered by precipitation separation, to separate 2,822 g of a fermented liquid (ethanol concentration: 2.16% by weight, sucrose content = 281 g, invert sugar content = 15 g).

### (2-2) Heating and cleaning step

The fermented liquid was transferred to a 5 L-beaker, and heated at 100°C for 10 minutes. Next, 0.085% by weight of slaked lime Ca(OH)₂ based on the weight of the squeezed juice was added thereto, to adjust pH and aggregate impurities. The aggregated impurities were filtrated with a filter, to separate 2,719 g of a cleaned liquid (ethanol concentration: 1.53% by weight, sucrose content = 277 g, invert sugar content = 15 g, pure sugar rate = 68.6%). Unlike Example 1, 19 g of ethanol was evaporated in the heating step.

### (3) Ethanol distillation and sugar liquid concentration step

The cleaned liquid was transferred to a 5 L-evaporator and heated under reduced pressure, and 42 g of evaporated ethanol was cooled and recovered. Thereafter, 2,104 mL of water was evaporated, to give 573 g of a concentrated sugar liquid (sucrose content = 277 g, invert sugar content = 15 g, pure sugar rate = 80.6%).

### (4) Crystallization step

A half of the sugar liquid was drawn out and further heated under reduced pressure, and concentrated to a degree of supersaturation of sucrose of 1.2. Thereafter, 29 g of seed crystals of sugar (particle size: 250 µm) were added thereto, and crystallization was carried out for about 3 hours, with adding the residual concentrated sugar liquid in small portions.

### (5) Raw sugar - molasses separation step

A mixture of the crystallized sugar and molasses was centrifuged in a perforated wall type centrifuge using a filter fabric with 50 to 100 µm mesh at 3,000 rpm for 20 minutes, to separate 186 g of sugar (sucrose recovery rate = 65%: excluding the amount of the added seed crystal) and 172 g of molasses (sucrose content = 97 g, invert sugar content = 12 g, pure sugar rate = 61.3%).

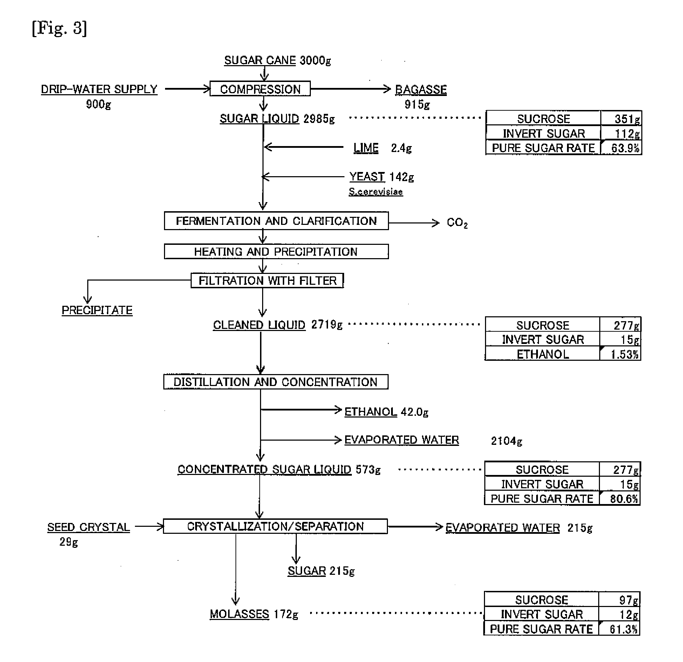

The result of the material balance of Comparative Example 1 is shown in Fig. 3.

## Claims

1. A method for producing sugar and ethanol comprising the steps of:
heating and cleaning a sugar liquid derived from a plant;
fermenting the cleaned liquid which has been adjusted to suitable temperature to selectively convert sugar components other than sucrose in the cleaned liquid into ethanol; and
concentrating the obtained fermented liquid.

2. The method for producing sugar and ethanol according to claim 1, wherein the fermentation is carried out using a yeast having no sucrose-degrading enzyme.

3. The method for producing sugar and ethanol according to claim 1, wherein the fermentation is carried out using a yeast of which sucrose-degrading enzyme gene is disrupted.

4. The method for producing sugar and ethanol according to claim 1, wherein the fermentation is carried out in the presence of a sucrose-degrading enzyme inhibitor.

5. The method for producing sugar and ethanol according to claim 1, wherein a crop as a raw material of the sugar is at least one kind selected from the group consisting of sugar cane, sugar beet, sugar palm, sugar maple and sorghum.

6. The method for producing sugar and ethanol according to any one of claims 1 to 5, wherein the cleaned liquid has a sucrose concentration of not less than 9% by weight.

7. The method for producing sugar and ethanol according to any one of claims 1 to 6, wherein the fermented liquid has a pure sugar rate of not less than 50%.

8. The method for producing sugar and ethanol according to any one of claims 1 to 7, wherein the concentration is conducted by distilling the fermented liquid under reduced pressure, and thereby, ethanol is separated and recovered from the fermented liquid.

9. The method for producing sugar and ethanol according to any one of claims 1 to 8 further comprising the step of crystallizing sugar from the concentrated sugar liquid obtained by concentrating the fermented liquid.
